(19)

Europäisches
Patentamt
European
Patent Office
Office européen
des brevets

(11) **EP 1 804 079 B1**

(12) **EUROPEAN PATENT SPECIFICATION**

(45) Date of publication and mention
of the grant of the patent:
**09.11.2011 Bulletin 2011/45**

(51) Int Cl.:
**G01S 15/89** (2006.01)     **A61B 8/12** (2006.01)
**A61B 8/08** (2006.01)     G01S 7/52 (2006.01)

(21) Application number: **06026944.6**

(22) Date of filing: **27.12.2006**

(54) **Ultrasonic diagnosis apparatus**

Ultraschalldiagnosegerät

Appareil de diagnostic à ultrasons

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **28.12.2005 JP 2005380209**

(43) Date of publication of application:
**04.07.2007 Bulletin 2007/27**

(73) Proprietor: **OLYMPUS MEDICAL SYSTEMS CORP.
Tokyo 151-0072 (JP)**

(72) Inventors:
• **Ikuma, Soichi
Shibuya-ku
Tokyo 151-0072 (JP)**
• **Kawashima, Tomonao
Shibuya-ku
Tokyo 151-0072 (JP)**

• **Komuro, Masahiko
Shibuya-ku
Tokyo 151-0072 (JP)**
• **Otani, Shuji
Shibuya-ku
Tokyo 151-0072 (JP)**
• **Adachi, Hideo
Shibuya-ku
Tokyo 151-0072 (JP)**

(74) Representative: **von Hellfeld, Axel
Wuesthoff & Wuesthoff
Patent- und Rechtsanwälte
Schweigerstrasse 2
81541 München (DE)**

(56) References cited:
**WO-A-96/25881          WO-A1-2005/092197
US-A- 6 048 312         US-A- 6 129 672
US-A- 2005 090 742      US-A1- 2003 028 107
US-A1- 2004 133 168     US-A1- 2005 137 478
US-B1- 6 186 949        US-B1- 6 733 458**

EP 1 804 079 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

1. Field of the Invention

The present invention relates to an ultrasonic diagnosis apparatus for creating an ultrasonic image from ultrasonic signals obtained by exchanging ultrasonic wave with the inside of a living body.

2. Description of the Related Art

An ultrasonic diagnosis apparatus has been widely spread in recent years that transmits ultrasonic wave to the inside of a living body and receives the reflected waves from a biological tissue to observe the state of the living body as an image since the condition within the living body can be observed in realtime.

When a tumor is found during the observation by using the ultrasonic diagnosis apparatus, the cells or tissue may be extracted to examine by observing an ultrasonic image thereof and sticking the tumor in order to determine whether the found tumor is benign or malignant.

When a puncture needle is used in this case, the puncture needle may project diagonally with respect to an ultrasonic scan plane, or the puncture needle may bend. Then, whether the end of the puncture needle has reached to the inside of a tumor or not cannot be checked, and the accurate diagnosis may not be performed as it is. Therefore, the puncture needle may be required to stick again.

In order to address this point, Japanese Unexamined Patent Application Publication No. 2005-58584 proposes an ultrasonic diagnosis apparatus in which the position of a puncture needle is estimated from volume data resulting from the detection of the position of the puncture needle by a position sensor and the three-dimensional scan of ultrasonic wave by using a two-dimensional transducer array so that the puncture needle can be displayed on an ultrasonic image.

Japanese Unexamined Patent Application Publication No. 2000-185041 proposes an ultrasonic diagnosis apparatus for estimating the position of a puncture needle based on the position having a larger Doppler signal from the volume data resulting from the application of slight vibrations to the puncture needle and the three-dimensional scan of ultrasonic wave by using a two-dimensional transducer array.

Japanese Unexamined Patent Application Publication No. 2004-208859 proposes an ultrasonic diagnosis apparatus for displaying the amount of displacement of a puncture needle from a scan plane based on the luminance information based on the reflected waves from the puncture needle from the volume data resulting from the three-dimensional scan of ultrasonic wave by using a two-dimensional transducer array,

However, though Japanese Unexamined Patent Application Publication No. 2005-58584 discloses that a position sensor is attached to a radio wave puncture needle or a hollow puncture needle for ethanol injection, it does not indicate the type of the position sensor, and the positional relationship between the position sensor and the puncture needle during the detection operation is not clear.

Japanese Unexamined Patent Application Publication No. 2000-185041 may require the sacrifice of the frame rate for performing three-dimensional scanning in Doppler mode, which may decrease the realtime characteristic during a centesis operation and may not be a realistic resolution. Furthermore, in the technology disclosed in the publication, slight vibrations must be continuously applied to the puncture needle, which complicates the construction of the apparatus.

According to Japanese Unexamined Patent Application Publication No. 2004-208859, the ultrasonic data includes many pixels having closer luminance values, which makes the accurate extraction of the puncture needle only difficult in reality. The technology disclosed in the publication has a problem that the actual path of insertion of the puncture needle cannot be checked since the puncture needle itself is not displayed though the amount of displacement of the puncture needle from the scanned surface is displayed.

The ultrasonic endoscope to be inserted into a body cavity for use has an insertion portion having more flexibility than an external ultrasonic probe, and the end part cannot be visually recognized. Therefore, it is significantly difficult to correct the position of the ultrasonic transducers and/or the orientation of the tip of the puncture needle. See document WO96/25881 A1 in this context.

SUMMARY OF THE INVENTION

Accordingly, it is an object of the present invention to provide an ultrasonic diagnosis apparatus which allows the secure display of the path of insertion of a puncture needle.

Briefly, according to an aspect of the present invention, there is provided an ultrasonic diagnosis apparatus including an ultrasonic probe for three-dimensionally scanning ultrasonic waves in a living body, the ultrasonic probe including an ultrasonic transducer and a therapeutic device channel in which a therapeutic device is insertable, the therapeutic device for insertion into the therapeutic device channel, position detecting means for detecting a position of the ultrasonic probe and a position of an end part of the therapeutic device, a volume data creating means for creating ultrasonic volume data, based on an ultrasonic signal captured by the ultrasonic probe, a tomographic plane selecting means for selecting a tomographic plane from the ultrasonic volume data, based on information on the position of the ultrasonic probe and information on the position of the end part of the therapeutic device detected by the position detecting means, and a display device for displaying the tomographic plane selected by the tomographic plane selecting means as a two-dimensional ultrasonic image during the scanning operation.

The above and other objects, features and advantages of the invention will become more clearly understood from the following description referring to the accompanying drawings.

BRIEF DESCRIPTION OF THE DRAWINGS

Fig. 1 is a diagram showing a configuration of an ultrasonic diagnosis apparatus according to a first embodiment of the present invention;

Fig. 2 is an enlarged perspective diagram showing a configuration of the end part of the ultrasonic endoscope according to the first embodiment;

Fig. 3 is a diagram showing the comparison between the position of a two-dimensional ultrasonic image extracted in accordance with an operation on a two-dimensional image select key and the end part of the ultrasonic endoscope;

Fig. 4 is a diagram showing an example in which the longitudinal axis of a puncture needle is rendered within a two-dimensional ultrasonic image on a monitor screen according to the first embodiment;

Fig. 5 is a diagram showing a state that the first point is designated by moving a cursor to the basal position of the puncture needle on a two-dimensional ultrasonic image on the monitor screen according to the first embodiment;

Fig. 6 is a diagram showing a state that the second point is designated by moving the cursor to the tip position of the puncture needle on a two-dimensional ultrasonic image on the monitor screen according to the first embodiment;

Fig. 7 is a diagram showing a state that the longitudinal axis of the puncture needle is displayed on a two-dimensional ultrasonic image on the monitor screen as a result of the designation of the first and second points according to the first embodiment;

Fig. 8 is a diagram showing a configuration of an ultrasonic diagnosis apparatus according to a second embodiment of the present invention; and

Fig. 9 is an enlarged diagram showing the tips of a puncture needle and stylet having a first transmitting coil.

DESCRIPTION OF THE PREFERRED EMBODIMENTS

With reference to drawings, embodiments of the present invention will be described below.

[First Embodiment]

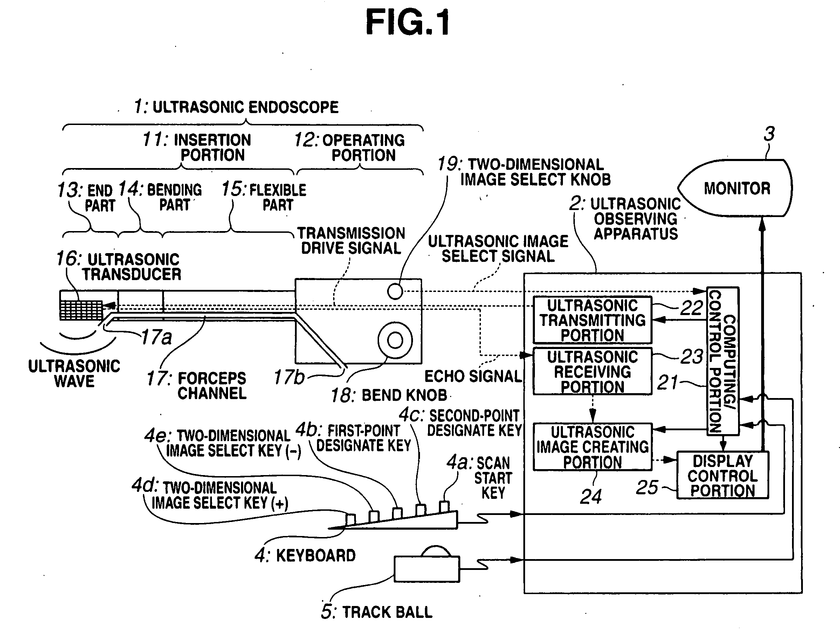

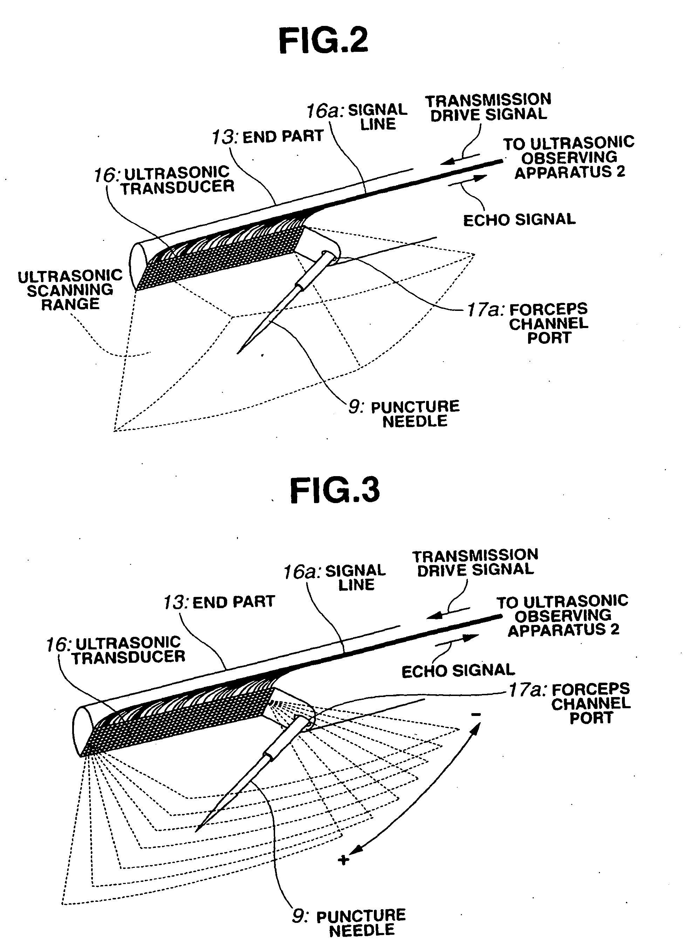

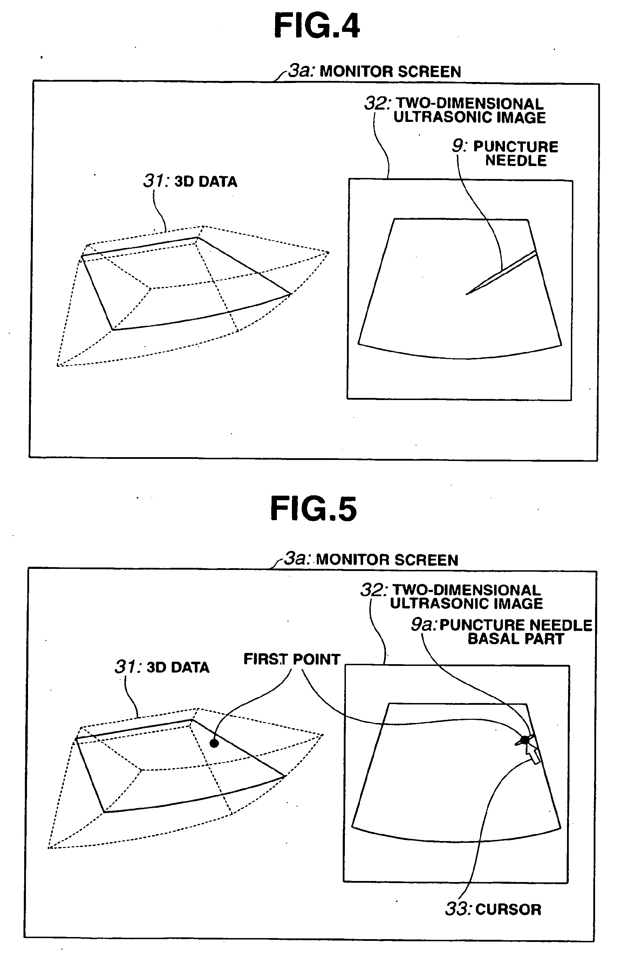

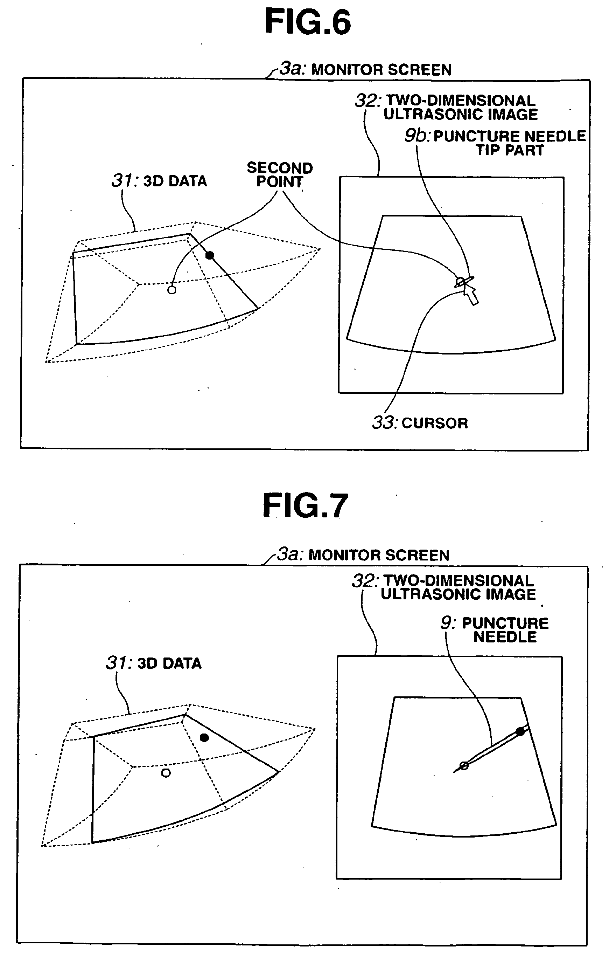

Figs. 1 to 7 show a first embodiment of the present invention, and Fig. 1 is a diagram showing a configuration of an ultrasonic diagnosis apparatus; Fig. 2 is an enlarged perspective diagram showing a configuration of the end part of the ultrasonic endoscope; Fig. 3 is a diagram showing the comparison between the position of a two-dimensional ultrasonic image extracted in accordance with an operation on a two-dimensional image select key and the end part of the ultrasonic endoscope; Fig. 4 is a diagram showing an example in which the longitudinal axis of a puncture needle is rendered within a two-dimensional ultrasonic image on a monitor screen; Fig. 5 is a diagram showing a state that the first point is designated by moving the cursor to the basal position of the puncture needle on a two-dimensional ultrasonic image on the monitor screen; Fig. 6 is a diagram showing a state that the second point is designated by moving the cursor to the tip position of the puncture needle on a two-dimensional ultrasonic image on the monitor screen; and Fig. 7 is a diagram showing a state that the longitudinal axis of the puncture needle is displayed on the two-dimensional ultrasonic image on the monitor screen as a result of the designation of the first and second points.

With reference to Fig. 1, a configuration of the ultrasonic diagnosis apparatus will be described. This embodiment employs an ultrasonic endoscope having multiple ultrasonic transducers aligned two-dimensionally as an ultrasonic probe.

The ultrasonic diagnosis apparatus includes an ultrasonic endoscope 1 functioning as an ultrasonic probe, an ultrasonic observing apparatus 2, a monitor 3, a keyboard 4 functioning as a tomographic place selecting unit and a manual selecting unit, and a trackball 5 functioning as a tomographic plane selecting unit and a manual selecting unit.

The ultrasonic endoscope 1 includes a long and narrow insertion portion 11, and an operating portion 12. The insertion portion 11 can be inserted to a body cavity of a patient. The operating portion 12 is connected to the rear end of the insertion portion 11 and is grasped by an operator to operate.

The insertion portion 11 has a rigid end part 13 at the end, a bending part 14, which is bendable, at the rear end of the end part 13 and a long flexible part 15 from the rear end of the bending part 14 to the front end of the operating portion 12.

The operating portion 12 has a bend knob 18 and a two-dimensional image select knob 19. The bend knob 18 is a bend-operation member for bending the bending part 14 in a desired direction in response to a rotating operation by an operator. The two-dimensional image select knob 19 is a tomographic plane selecting unit and manual selecting unit for selecting a two-dimensional ultrasonic image to be displayed on the monitor 3. The two-dimensional image select knob 19 is configured to be rotatable in two of clockwise and counter clockwise directions and outputs an ultrasonic image select signal in accordance with an operation to a computing/control portion 21, which will be described later, of the ultrasonic observing apparatus 2.

The ultrasonic endoscope 1 further includes a forceps channel 17 in the longitudinal direction from the operating portion 12 to the end part 13. The forceps channel 17 is a therapeutic device channel with a hollow tube providing a tunnel structure. The forceps channel 17 has forceps channel ports 17a and 17b, which are open to the end part 13 and the operating portion 12, respectively. The forceps channel 17 may internally hold a therapeutic device such as a puncture needle.

With reference to Fig. 2, the configuration of the end part 13 will be described below.

The end part 13 includes multiple ultrasonic transducers 16. The multiple ultrasonic transducers 16, more specifically, are aligned in a two-dimensional plane form having a transducer array in parallel with the direction of insertion and a transducer array perpendicular thereto, which form a two-dimensional array. Each of the ultrasonic transducers 16 connects to a signal line 16a. Each of the signal lines 16a connects to the ultrasonic observing apparatus 2. A pulse-shaped transmission drive signal for driving the ultrasonic transducers 16 and an echo signal from the ultrasonic transducers 16 are exchanged through the signal lines 16a. In this case, each of the ultrasonic transducers 16 is driven, as described later, to obtain an echo from a three-dimensional space in a specific area.

The forceps channel port 17a on the end part 13 side of the forceps channel 17 opens about the center axis having a predetermined angle from the axis of insertion so that a puncture needle 9 (see Fig. 2), which is a therapeutic device, for example, can project within the range of the ultrasonic scan.

Notably, the end part 13 has an illumination window and an observation window, not shown. The illumination window is used for irradiating illumination light into the body cavity. The observation window has an optical lens for observing an illuminated subject.

Referring back to Fig. 1, the ultrasonic observing apparatus 2 includes the computing/control portion 21, which is a tomographic plane selecting unit, an ultrasonic transmitting portion 22, an ultrasonic receiving portion 23, an ultrasonic image creating portion 24, which is a volume data creating unit, and a display control portion 25, which is a tomographic plane selecting unit.

The computing/control portion 21 functions as a control unit for controlling operations of components within the ultrasonic observing apparatus 2 including the ultrasonic transmitting portion 22, ultrasonic image creating portion 24 and display control portion 25 and also functions as a computing unit for performing required computing, for example.

The ultrasonic transmitting portion 22 transmits a pulse-shaped transmission drive signal as described above for driving the ultrasonic transducers 16.

The ultrasonic receiving portion 23 receives an echo signal as described above from the ultrasonic transducers 16.

The ultrasonic image creating portion 24 creates ultrasonic volume data (called 3D data, hereinafter), which includes three-dimensional ultrasonic image data, based on an echo signal received from the ultrasonic receiving portion 23.

The display control portion 25 is a display control unit for controlling to display the 3D data created by the ultrasonic image creating portion 24 on the monitor 3 under the control of the computing/control portion 21 and controlling to extract and display a two-dimensional ultrasonic image from the 3D data on the monitor 3.

The ultrasonic observing apparatus 2 connects to the keyboard 4. The keyboard 4 includes a scan start key 4a, a first-point designate key 4b, a second-point designate key 4c, a two-dimensional image select key (+) 4d and a two-dimensional image select key (-) 4e.

The scan start key 4a is for starting ultrasonic scan by the ultrasonic transducers 16.

The first-point designate key 4b is, for example, for designating the basal side of the part of the puncture needle 9 projecting from the forceps channel port 17a as a first point, as described later.

The second-point designate key 4c is, for example, for designating the tip side of the part of the puncture needle 9 projecting from the forceps channel port 17a as a second point, as described later.

The two-dimensional image select key (+) 4d is for selecting a two-dimensional ultrasonic image to be displayed on the monitor 3.

The two-dimensional image select key (-) 4e is also for selecting a two-dimensional ultrasonic image to be displayed on the monitor 3 and performs the designation in the opposite direction of that of the two-dimensional image select key (+) 4d.

The ultrasonic observing apparatus 2 further connects to the trackball 5. The trackball 5 is a pointing device for moving a cursor 33 displayed on a monitor screen 3a of the monitor 3 (see Figs. 5 and 6).

The ultrasonic observing apparatus 2 further connects to the monitor 3. The monitor 3 is a display unit (display apparatus) for displaying the output from the ultrasonic observing apparatus 2.

Next, operations of the ultrasonic diagnosis apparatus as shown in Figs. 1 and 2 will be described.

In Fig. 1, the broken line, thick solid line and solid line indicate a flow of a signal/data relating to ultrasonic wave, a flow of a signal/data relating to a final display image and a flow of signal/data relating to control, respectively.

First of all, an operation of the entire ultrasonic diagnosis apparatus will be described schematically.

When the scan start key 4a of the keyboard 4 is pressed, a pulse-voltage-shaped transmission drive signal from the ultrasonic transmitting portion 22 to the ultrasonic transducers 16 in the end part 13 of the ultrasonic endoscope 1 under the control of the computing/control portion 21 within the ultrasonic observing apparatus 2. In this case, the computing/control portion 21 delays excitation signals (that is, transmission drive signals) so as to differentiate the times when transmission drive signals reach the ultrasonic transducers 16.

Partial or multiple ultrasonic transducers 16 of the multiple ultrasonic transducers 16 in the ultrasonic transducer array receive and convert pulse-voltage-shaped excitation signals from the ultrasonic transmitting portion 22 to ultrasonic wave, which is compression wave of a medium. The ultrasonic wave excited by the ultrasonic transducers 16 overlap within a subject body to form one ultrasonic beam (the computing/control portion 21 delays the transmission drive signals as described above to form the one ultrasonic beam). The ultrasonic beam generated in this way is irradiated to the outside of the ultrasonic endoscope 1 and scans the inside of a subject three-dimensionally. The reflected wave from the inside of the subject returns to the ultrasonic transducers 16 through the reverse path of that of the ultrasonic beam. The ultrasonic transducers 16 convert the reflected wave to an electric echo signal and transmit the echo signal to the ultrasonic receiving portion 23 within the ultrasonic observing apparatus 2 through the reverse path of that of the excitation signal.

The ultrasonic receiving portion 23 amplifies and transmits the received echo signal to the ultrasonic image creating portion 24.

The ultrasonic image creating portion 24 phases and adds the amplified echo signals and creates the 3D data under the control of the computing/control portion 21.

The display control portion 25 extracts and outputs the two-dimensional ultrasonic image from the 3D data created by the ultrasonic image creating portion 24 to the monitor 3 under the control of the computing/control portion 21.

In other words, when an operator operates the two-dimensional image select knob 19 of the operating portion 12 of the ultrasonic endoscope 1, the two-dimensional image select key 4d or 4e on the keyboard 4 or the trackball 5, the computing/control portion 21 within the ultrasonic observing apparatus 2 controls the display control portion 25 to designate the plane to extract the two-dimensional ultrasonic image from the 3D data. Thus, the display control portion 25 extracts the plane designated by the computing/control portion 21 to create and output the two-dimensional ultrasonic image to the monitor 3. Hence, the operator can display the tomographic plane rendered by the puncture needle 9 and can perform accurate centesis while observing the image of the puncture needle 9.

Next, how to designate the plane to extract the two-dimensional ultrasonic image will be described.

Initially, when the puncture needle 9 projects straight, without bending, from the forceps channel port 17a toward the exit, the computing/control portion 21 controls the display control portion 25 to display the tomographic plane of the puncture needle 9 and extract the two-dimensional ultrasonic image position (called initial two-dimensional image position, hereinafter) perpendicular to the transducer array plane.

Then, in response to the clockwise rotational operation of the two-dimensional image select knob 19 disposed at the operating portion 12 of the ultrasonic endoscope 1 or the press of the two-dimensional image select key (+) 4d on the keyboard 4, the computing/control portion 21 controls the display control portion 25 to extract the two-dimensional ultrasonic image position resulting from the rotation of the initial two-dimensional image position about the center line of the axis of insertion as the rotational axis by the angle in accordance with the amount of operation in the + direction in Fig. 3 (in the clockwise direction when the basal side is viewed from the tip side through the axis of insertion).

On the other hand, in response to the counter-clockwise rotational operation of the two-dimensional image select knob 19 disposed at the operating portion 12 of the ultrasonic endoscope 1 or the press of the two-dimensional image select key (-) 4e on the keyboard 4, the computing/control portion 21 controls the display control portion 25 to extract the two-dimensional ultrasonic image position resulting from the rotation of the initial two-dimensional image position about the center line of the axis of insertion as the rotational axis by the angle in accordance with the amount of operation in the - direction in Fig. 3 (in the counter-clockwise direction when the basal side is viewed from the tip side through the axis of insertion).

In order to display an arbitrary tomographic plane as a two-dimensional ultrasonic image during the performance of the processing as described above, processing may be performed as follows.

First of all, as shown in Fig. 5 or 6, the monitor screen 3a of the monitor 3 can display a 3D data 31 and a two-dimensional ultrasonic image 32 in a line. The monitor screen 3a can further display a cursor 33 functioning as a pointer.

In this configuration, an operator operates the trackball 5 at a desired time to display the cursor 33 on the two-dimensional ultrasonic image 32 on the monitor screen 3a and presses the first point designate key 4b on the keyboard 4. Then, the computing/control portion 21 controls the display control portion 25 to display a red point, for example, at the position designated by the cursor 33 on the two-dimensional ultrasonic image 32 and also display the red point at the corresponding position on the 3D data 31. If the second point, which will be described later, has been already designated until then, the computing/control portion 21 controls the display control portion 25 to extract the plane through the first and second points and in perpendicular to the transducer plane as the two-dimensional ultrasonic image 32.

Under the condition that the first point is designated, an operator may operate the trackball 5 to move the cursor 33 to the position outside of the two-dimensional ultrasonic image 32 on the monitor screen 3a and may then press the first point designate key 4b on the keyboard 4. Then, the computing/control portion 21 controls the display control portion 25 to cancel the designation of the first point and return to the condition that the red points displayed on the two-dimensional ultrasonic image 32 and 3D data 31 are not displayed.

Similarly, during the performance of the processing, an operator may operate the trackball 5 to display the cursor 33 on the two-dimensional ultrasonic image 32 on the monitor screen 3 a and may then press the second point designate key 4c on the keyboard 4. Then, the computing/control portion 21 controls the display control portion 25 to display a green point, for example, at the position designated by the cursor 33 on the two-dimensional ultrasonic image 32 and also display the green point at the corresponding position on the 3D data 31. If the first point, which has been described above, has been already designated until then, the computing/control portion 21 controls the display control portion 25 to extract the plane through the first and second points and in perpendicular to the transducer plane as the two-dimensional ultrasonic image 32.

Under the condition that the second point is designated, the operator may operate the trackball 5 to move the cursor 33 to the position outside of the two-dimensional ultrasonic image 32 on the monitor screen 3a and may then press the second point designate key 4c on the keyboard 4. Then, the computing/control portion 21 controls the display control portion 25 to cancel the designation of the second point and to return to the condition that the green points displayed on the two-dimensional ultrasonic image 32 and 3D data 31 are not displayed.

Under the condition that the two points (that is, the first and second points) are designated, when the two-dimensional image select knob 19 in the operating portion 12 of the ultrasonic endoscope 1 is operated to rotate, or when the two-dimensional image select key 4d or 4e on the keyboard 4 is pressed, the computing/control portion 21 controls the display control portion 25 to extract the two-dimensional ultrasonic image 32 resulting from the rotation about the straight line through the first and second points as the axis of rotation by the angle in accordance with the amount of operation.

The operation for extracting and displaying a two-dimensional ultrasonic image will be described in more detail with reference to an example in which the longitudinal axis of the puncture needle 9 is rendered within the two-dimensional ultrasonic image 32 on the monitor screen 3a, as shown in Fig. 4.

First, when an operator may press the scan start key 4a on the keyboard 4, the 3D (three-dimensional) scan is started, and the monitor screen 3a displays the image of the 3D data 31 and the two-dimensional ultrasonic image 32. Here, the two-dimensional ultrasonic image 32 displays the position at the initial two-dimensional image position as described above.

Next, an operator may move the end part 13 of the ultrasonic endoscope 1 to the vicinity of the part to stick and causes a small amount of the puncture needle 9 to project from the forceps channel port 17a through the forceps channel 17.

Then, the operator may operate the two-dimensional image select knob 19 in the operating portion 12 of the ultrasonic endoscope 1 or the two-dimensional image select key 4d or 4e on the keyboard 4 to display the two-dimensional ultrasonic image 32 displaying the longitudinal direction of the puncture needle 9 as shown in Fig. 5.

Here, when the direction of projection of the puncture needle 9 and the direction of the axis of insertion of the ultrasonic endoscope 1 are twisted, the longitudinal axis of the puncture needle 9 is not displayed well on the two-dimensional ultrasonic image 32.

In this case, an operation may be performed for designating an arbitrary tomographic plane as the two-dimensional ultrasonic image 32.

In other words, the two-dimensional image select knob 19 or two-dimensional image select key 4d or 4e is first operated to display the basal part 9a of the puncture needle 9 on the two-dimensional ultrasonic image 32.

Next, the trackball 5 is operated to display the cursor 33 at the position of a basal part 9a of the puncture needle 9 on the two-dimensional ultrasonic image 32 on the monitor screen 3a as shown in Fig. 5, and the first point designate key 4b on the keyboard 4 is pressed.

Then, the two-dimensional image select knob 19 or two-dimensional image select key 4d or 4e is operated to display a tip part 9b of the puncture needle 9 on the two-dimensional ultrasonic image 32 as shown in Fig. 6.

Then, the trackball 5 is operated to display the cursor 33 at the position of the tip part 9b of the puncture needle 9 on the two-dimensional ultrasonic image 32 on the monitor screen 3a as shown in Fig. 6, and the second point designate key 4c on the keyboard 4 is pressed.

With the above operation, the longitudinal axis of the puncture needle 9 is displayed on the two-dimensional ultrasonic image 32 as shown in Fig. 7.

Here, in order to check the positional relationship with a surrounding organ or when the puncture needle 9 bends, the two-dimensional image select knob 19 or two-dimensional image select key 4d or 4e is operated to rotate the two-dimensional ultrasonic image 32 about the straight line through the first and second points to display the surrounding organ or the bending state of the puncture needle 9 on the monitor 3 for checking.

After that, the centesis is performed more deeply onto an affected part by checking the puncture needle 9 on the two-dimensional ultrasonic image 32 at the same time. During the performance of the centesis, the two points (that is, the first and second points) may be designated as described above again, and the angle of rotation may be adjusted to change the tomographic plane to be displayed when the position of the end part 13 of the ultrasonic endoscope 1 is moved or the direction of the puncture needle 9 is changed. The display of the arbitrary tomographic plane may be cancelled by operating the trackball 5 to move the cursor 33 to the position outside of the two-dimensional ultrasonic image 32 on the monitor screen 3a and pressing the first point designate key 4b or second point designate key 4c on the keyboard 4. Then, in order to check the puncture needle 9, the two-dimensional image select knob 19 may be rotated, and the two-dimensional ultrasonic images 32 displaying the tip part 9b of the puncture needle 9 may be sequentially selected.

When the puncture needle 9 bends, the two points near the tip of the puncture needle 9 may be designated to display the path through which the tip of the puncture needle 9 may pass.

The end part 13 may be checked all the time on a real ultrasonic tomographic image by rotating the two-dimensional image select knob 19 and sequentially selecting the two-dimensional ultrasonic image 32 displaying the tip part of the puncture needle 9.

According to the first embodiment, the tip of the puncture needle 9 can be checked securely on the two-dimensional ultrasonic image 32 not only when the puncture needle 9 projects straight from the forceps channel port 17a but also when the puncture needle 9 projects diagonally from the forceps channel port 17 a or the puncture needle 9 itself bends. Thus, the reach of the tip of the puncture needle 9 to the inside of a tumor can be confirmed, which can improve the accuracy of the diagnosis. Furthermore, the operation can be continued even with the puncture needle 9 bent, which can improve the cost efficiency and reduce the examination time by eliminating the necessity for replacing the bent puncture needle 9 and sticking the new one again.

Unlike an external probe, the ultrasonic endoscope 1 is generally grasped by both hands so that the load to be imposed on an operator can be reduced by configuring the selection of the two-dimensional ultrasonic image 32 to be displayed on the monitor 3 to be performed by using the two-dimensional image select knob 19 in the operating portion 12. The centesis is performed by an operator with one hand operating the operating portion 12 and the other hand operating the puncture needle 9. The two-dimensional image select knob 19 in the operating portion 12 eliminates the necessity for an assistant to display the two-dimensional ultrasonic image 32 of the tip of the puncture needle 9, which improves the operability for centesis.

Since the two-dimensional ultrasonic image 32 to be displayed may be selected manually, a desired image can be displayed more securely than that of a device for automatically displaying an image. The surrounding organ of the puncture needle can be checked by displaying a two dimensional ultrasonic image of the plane except for the place having the puncture needle. This can further simplify the configuration and reduce the size of the apparatus.

The technology of this embodiment displays an echo from the puncture needle 9 displayed on a real ultrasonic image. Thus, this technology provides more accurate positional relationship with a real surrounding organ than a conventional technology, which displays the position of the puncture needle 9 estimated based on data obtained from a sensor, for example, on an ultrasonic tomographic image in a superimposed manner. Thus, an affected part can be securely stuck, and the cells or tissue of the affected part can be securely extracted.

[Variation Examples]

Having employed the ultrasonic endoscope 1 having a two-dimensional array including the multiple ultrasonic transducers 16 in a two-dimensional plane form as an ultrasonic probe, the invention is not limited thereto. For example, an ultrasonic probe having a two-dimensional array including the multiple ultrasonic transducers 16 in a two-dimensional curved form may be used instead. More specifically, a two-dimensional array including the multiple ultrasonic transducers 16 along the outer edge of the cylindrical end part 13 may be used, for example. Alternatively, an ultrasonic probe may be used which scans three-dimensionally by mechanically moving an array of transducers aligned in one-dimensionally. The ultrasonic probe is not limited to an ultrasonic endoscope.

The keyboard 4 and trackball 5 have been employed as units for externally controlling the ultrasonic observing apparatus 2 for the illustration purpose only. For example, a mouse and/or a joystick may be used as a unit for selecting an item under a menu on a screen.

Having described that, when the two-dimensional ultrasonic image 32 is selected, the two-dimensional ultrasonic image 32 is displayed resulting from the rotation about the center line of the axis of insertion by the angle in accordance with the amount of operation, the invention is not limited thereto. The two-dimensional ultrasonic image 32 may be displayed resulting from the rotation of the initial two-dimensional image position about the straight line through the center of the forceps channel port 17a in the end part 13 of the ultrasonic endoscope 1. The adoption of this configuration allows the easier selection of the two-dimensional ultrasonic image 32 rendering the longitudinal axis of the puncture needle 9 since the forceps channel port 17a fixing the position of the puncture needle 9 always exists on the two-dimensional ultrasonic image 32.

In addition, having described that the two-dimensional ultrasonic image 32 may be selected by two types of the unit for displaying the two-dimensional ultrasonic image 32 by simultaneously rotating it about the center line of the axis of insertion by the angle in accordance with the amount of operation and the unit for displaying an arbitrary two-dimensional ultrasonic image 32 by designating two points and the angle of rotation. However, the invention is not limited thereto, and other units may be used instead as follows.

An example thereof is a unit for selecting and displaying an arbitrary two-dimensional ultrasonic image 32 including designated three points.

Another example thereof is a unit for rotating and displaying the initial two-dimensional image position about orthogonal three straight lines through the center position of the opening of the forceps channel port 17a of the end part 13 of the ultrasonic endoscope 1. This allows the rotation and display of the initial two-dimensional image about the axes of coordinates defined by handling the center position of the opening of the forceps channel port 17a as the origin. The adoption of this unit allows the display of the longitudinal axis of the puncture needle 9 on the two-dimensional ultrasonic image 32 by designating only three angles of rotation even when the direction of projection of the puncture needle 9 is displaced in any direction. In this case, providing three devices for inputting the angle of rotation, such as a knob and a button, in the operating portion 12 of the ultrasonic endoscope 1 allows easy display of the longitudinal axis of the puncture needle 9 on the two-dimensional ultrasonic image 32 only by a hand operation.

[Second Embodiment]

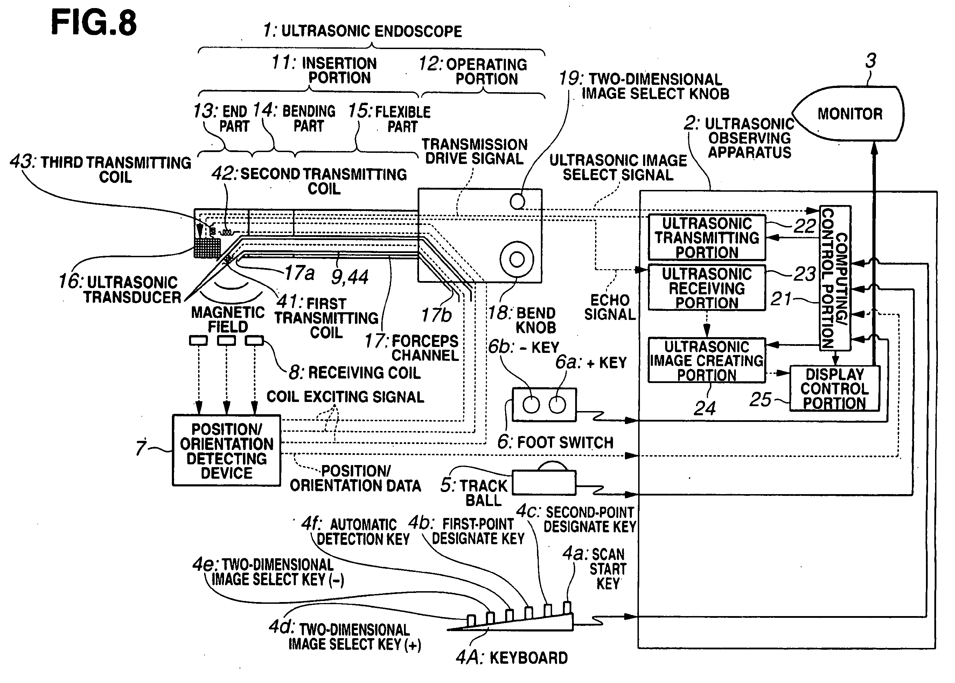

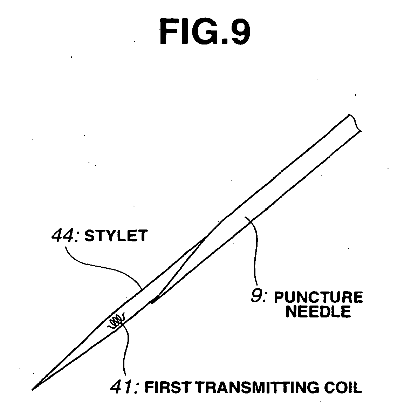

Figs. 8 and 9 show a second embodiment of the preset invention. Fig. 8 is a diagram showing a configuration of an ultrasonic diagnosis apparatus, and Fig. 9 is an enlarged diagram showing the tip of a stylet having a puncture needle and a first transmitting coil.

According to the second embodiment, the same reference numerals are given to the same components as those of the first embodiment, and the repetitive description thereof will be omitted herein. Mainly, differences will be only described.

According to this embodiment, the ultrasonic diagnosis apparatus of the second embodiment is different from that of the first embodiment shown in Fig. 1 in following points.

First of all, referring to Fig. 9, the tips of the puncture needle 9 and stylet 44 will be described.

The puncture needle 9 is a therapeutic device to be stuck into a living body and has a hollow structure allowing the suction of cells, for example, or injection of ethanol, for example.

The puncture needle 9 has a hollow part holding the stylet 44, which is a therapeutic device having a sharp needle form at the tip. The tip part of the stylet 44 internally includes a first transmitting coil 41 functioning as a position detecting unit and magnetic sensor and having the direction of the axis of wiring agreed with the direction of the axis of the stylet 44. The first transmitting coil 41 connects to a position/orientation detecting device 7, which will be described later, through a signal line, as shown in Fig. 8.

The ultrasonic diagnosis apparatus of this embodiment includes a foot switch 6, which functions as a tomographic plane selecting unit and a manual selecting unit, the position/orientation detecting device 7, and a receiving coil 8 functioning as the position detecting unit and a magnetic sensor. The configuration of a keyboard 4A functioning as a tomographic plane selecting unit and a manual selecting unit is slightly different from that of the keyboard 4 of the first embodiment.

The end part 13 of the ultrasonic endoscope 1 internally includes a second transmitting coil 42 and a third transmitting coil 43, both of which functioning as a position detecting unit and a magnetic sensor. The second transmitting coil 42 of them is provided with the direction of axis of wiring agreed with the direction of axis of insertion of the ultrasonic endoscope 1 and also agreed with the longitudinal direction of the plane of the ultrasonic transducers 16 in a two-dimensional array. The third transmitting coil 43 has the direction of axis of wiring orthogonal to the direction of axis of wiring of the second transmitting coil 42 and perpendicular to the plane of the ultrasonic transducers 16 in a two-dimensional array. The second transmitting coil 42 and third transmitting coil 43 are connected to the position/orientation detecting device 7 through respective signal lines.

The position/orientation detecting device 7 is connected to the first to third transmitting coils 41 to 43, as described above, and functions as a position/orientation detecting unit for outputting coil excitation signals to the first to third transmitting coils 41 to 43. The position/orientation detecting device 7 is further connected to multiple receiving coils 8. The multiple receiving coils 8 are spatially fixed with the different directions of the axis of wiring. The position/orientation detecting device 7 receives current generated from the receiving coils 8 in response to a change in magnetic field, calculates position/orientation data and transmits the result to the computing/control portion 21 of the ultrasonic observing apparatus.

The foot switch 6 includes a + key 6a and - key 6b, each of which is a button to be operated by a foot. The foot switch 6 is connected to the computing/control portion 21 of the ultrasonic observing apparatus 2.

The keyboard 4A includes the scan start key 4a, first point designate key 4b, second point designate key 4c, two-dimensional image select key (+) 4d and two-dimensional image select key (-) 4e and further includes an automatic detection key 4f.

The rest of the configuration of the ultrasonic diagnosis apparatus is substantially the same as that of the ultrasonic diagnosis apparatus of the first embodiment.

Next, operations of the ultrasonic diagnosis apparatus will be described.

The operations of the ultrasonic diagnosis apparatus of this embodiment are different from the operations of the ultrasonic diagnosis apparatus of the first embodiment in the operation of the stylet 44, the operation of the position/orientation detecting device 7, the operation for designating the plane to extract the two-dimensional ultrasonic image 32 from the 3D data 31 by the computing/control portion 21 and the operation by the foot switch 6. Only the differences will be mainly described below.

In Fig. 8, the thick broken line, broken line, thick solid line and solid line indicate a flow of a signal/data relating a position, a flow of a signal/data relating to ultrasonic wave, a flow of a signal/data relating to a final display image and a flow of signal/data relating to control, respectively.

First of all, the centesis and suction, for example, may be performed as follows. That is, the puncture needle 9 and the style 44 are integrally stack into an affected part with the stylet 44 projected from the puncture needle 9. During the centesis operation, the tissue on the centesis path to the affected part is prevented from incorporating in the puncture needle 9 since the stylet 44 is held within the hollow part of the puncture needle 9. After that, the stylet 44 is released from the puncture needle 9 to suck cells, for example, or inject ethanol, for example.

Next, the operation of the position/orientation detecting device 7 by using the transmitting and receiving coils is performed as follows.

The position/orientation detecting device 7 excites, with different frequencies, the first transmitting coil 41 within the stylet 44, the second transmitting coil 42 in the end part 13 of the ultrasonic endoscope 1 and the third transmitting coil 43 in the end part 13.

The receiving coil 8 detects the alternate magnetic fields from the first to third transmitting coils 41 to 43 and converts and outputs the detected magnetic fields to position electric signals to the position/orientation detecting device 7.

The position/orientation detecting device 7 separates the position electric signals inputted from the receiving coil 8 for each frequency so that the transmitting coil having the magnetic field from which the given position electric signal is detected can be identified. Then, the position/orientation detecting device 7 calculates the position/orientation data of the transmitting coil based on each of the separated position electric signals and outputs the calculated position/orientation data to the computing/control portion 21 of the ultrasonic observing apparatus 2.

Here, according to this embodiment, the origin O is defined on the receiving coil 8, and the orthogonal coordinate axes O-xyz and the normal orthogonal basebands (unit vectors of axis directions) i, j and k (where normal letters are used for the notation of vectors instead of thick letters. The same is true hereinafter.) are fixed in a real space for examining a subject by an operator.

In this case, based on the position electric signals, the position/orientation detecting device 7 calculates, as a function of time t, and outputs to the computing/control portion 21 of the ultrasonic observing apparatus 2 the position/orientation data of the transmitting coils 41 to 43 as:

Direction components to the orthogonal coordinate axes O-xyz of a position vector $OC_1(t)$ of a position $C_1(t)$ of the first transmitting coil 41,
Direction components to the orthogonal coordinate axes O-xyz of a unit vector $V_1(t)$ indicating the axis direction of the wire of the first transmitting coil 41,
Direction components to the orthogonal coordinate axes O-xyz of a position vector $OC_2(t)$ of a position $C_2(t)$ of the second transmitting coil 42,
Direction components to the orthogonal coordinate axes O-xyz of a unit vector $V_2(t)$ indicating the axis direction of the wire of the second transmitting coil 42,
Direction components to the orthogonal coordinate axes O-xyz of a position vector $OC_3(t)$ of a position $C_3(t)$ of the third transmitting coil 43, and
Direction components to the orthogonal coordinate axes O-xyz of a unit vector $V_3(t)$ indicating the axis direction of the wire of the third transmitting coil 43.

When the automatic detection key 4f on the keyboard 4A is pressed here, the computing/control portion 21 performs an operation, which is different from that of the first embodiment, as follows:

First of all, according to this embodiment, the origin O' is defined at the center position of a two-dimensional array including the ultrasonic transducers 16, and the orthogonal coordinate axes O'-x'y'z' and the normal orthogonal basebands (unit vectors of axis directions) i', j' and k' are fixed in a real space for examining a subject by an operator as expressed by:

$$i'=V_2(t)$$
$$j'=V_3(t)$$
$$k'= V_2(t) \times V_3(t) \qquad [EQ1]$$

where the symbol "$\times$" in the right side expressing k' expresses the outer product.

In this case, the position of the origin O' can be calculated based on the $C_2(t)$ and $C_3(t)$ outputted from the position/orientation detecting device 7 and the designed relative positions of the two-dimensional array including the ultrasonic transducers 16 and the second and third transmitting coils 42 and 43 within the end part 13 of the ultrasonic endoscope 1.

Next, the $C_1(t)$ and $V_1(t)$ outputted from the position/orientation detecting device 7 are converted in coordinates to $C_1'(t)$ and $V_1'(t)$, which are values in the O'-x'y'z' coordinates.

Then, the computing/control portion 21 controls the display control portion 25 to extract the two-dimensional ultrasonic image 32 including $C_1'(t)$ and $V_1'(t)$ and being in perpendicular to the transducer plane from the 3D data 31.

The computing/control portion 21 repeats a series of these steps every time position/orientation data is inputted from the position/orientation detecting device 7.

When the automatic detection key 4f on the keyboard 4A is pressed again, the display control portion 25 is controlled to fix the position of the two-dimensional ultrasonic image 32 to be extracted from the 3D data 31 at the position when pressed. By handling the position as the initial two-dimensional image position, the ultrasonic diagnosis apparatus performs the same operation as that of the first embodiment.

The + key 6a of the foot switch 6 is used for performing the same operations as those of the two-dimensional image select key (+) 4d on the keyboard 4A, and the - key 6b of the foot switch 6 is used for performing the same operations as those of the two-dimensional image select key (-) 4e on the keyboard 4A.

The other operations in this embodiment are the same as those of the first embodiment.

The second embodiment can provide substantially the same effects as those of the first embodiment and can always and automatically display the two-dimensional ultrasonic image 32 rendering the longitudinal axis of the puncture needle 9, which eliminates the necessity for manual positioning and can reduce the load on an operator and the examination time.

Since the stylet 44 includes the transmitting coils, a position sensor can be internally provided in the hollow puncture needle 9 without preventing a sticking operation.

Since the position can be changed manually after the two-dimensional ultrasonic image 32 rendering the longitudinal axis of the puncture needle 9 is automatically displayed, the puncture needle 9 can be securely rendered by fine adjustments even when an operation for moving the puncture needle 9 having released the stylet 44 within the affected part in order to improve the efficiency of cell or tissue extraction. Furthermore, the puncture needle 9 can be securely rendered by fine adjustments even when the accuracy of the sensor decreases due to the poor surrounding atmosphere of the magnetic field.

Furthermore, the two-dimensional ultrasonic image 32 may be selected by a foot since the foot switch 6 is provided. In this case, the two-dimensional image select knob 19 may be omitted from the operating portion 12, which can reduce the weight of the operating portion 12 of an ultrasonic probe without decreasing the effect that operations can be performed even when both hands are full.

[Variation Examples]

In the description above, the transmitting coils 41 to 43 for exciting alternate magnetic fields are provided in the end part 13 of the ultrasonic endoscope 1 and the tip of the stylet 44, and the receiving coil 8 for detecting an alternate magnetic field and outputting a position electric signal is placed at an external predetermined position of the ultrasonic endoscope 1. However, the invention is not limited thereto, but the transmitting coils 41 to 43 may be placed at external predetermined positions of the ultrasonic endoscope 1, and the receiving coil 8 may be provided in the end part 13 of the ultrasonic endoscope 1 and the tip of the stylet 44. Even in this configuration, the position/orientation detecting device 7 can also calculate and output position/orientation data of the transmitting coils based on the position electric signal outputted by the receiving coil 8.

Having described that one coil is provided in the stylet 44, two coils, for example, may be provided. In this case, the two-dimensional ultrasonic image 32 including the positions of the coils and being in parallel with the axis direction vector of the wire of any one coil may be extracted from the 3D data 31. Thus, even when the puncture needle 9 is bent, the two-dimensional ultrasonic image 32 rendering the longitudinal axis of the puncture needle 9 can be displayed.

Furthermore, according to this embodiment, the same variation examples as those of the first embodiment are applicable in addition.

[Appendices]

The embodiments of the invention as described in detail above provide following configurations:

[Appendix 1]

An ultrasonic diagnosis apparatus comprising:

an ultrasonic probe for three-dimensionally scanning ultrasonic wave in a living body;
volume data creating means for creating ultrasonic volume data based on an ultrasonic signal captured by the ultrasonic probe;
tomographic plane selecting means for selecting a tomographic plane from the ultrasonic volume data; and
a display device for displaying the tomographic plane selected by the tomographic plane selecting means as a two-dimensional ultrasonic image during the scanning operation.

In this case, a tomographic plane may be selected from ultrasonic volume data during a scanning operation, and the selected tomographic image may be displayed as a two-dimensional ultrasonic image. Thus, the tip of the puncture needle can be checked securely on the two-dimensional ultrasonic image even when the puncture needle projects diagonally or the puncture needle bends. Hence, the reach of the tip of the puncture needle of the inside of a tumor can be confirmed, which can improve the accuracy of the diagnosis. Furthermore, the cost efficiency can be improved by eliminating the necessity for replacing the bent puncture needle and sticking the new one again.

[Appendix 2]

The ultrasonic diagnosis apparatus according to Appendix 1,
wherein the tomographic plane selecting means has manual selecting means for inputting the selection of a tomographic plane manually.
In this case, since a tomographic plane to be displayed may be selected and inputted manually, a desired tomographic can be selected more securely than that of a device for automatically selecting by using a sensor, for example, which can reduce the size and simplify of the configuration of the apparatus. In addition, the display from the angle desired to display by an operator can be implemented more securely.

[Appendix 3]

The ultrasonic diagnosis apparatus according to Appendix 2,
wherein the ultrasonic probe has an operating portion for operations by the hand; and
the manual selecting means is provided in the operating portion of the ultrasonic probe.
In this case, since a tomographic plane may be selected and inputted by the hands through the operating portion, a two-dimensional ultrasonic image to be displayed on the display apparatus can be selected by grasping the ultrasonic probe with both hands even when the ultrasonic probe is of the type to be generally grasped by both hands (such as an ultrasonic endoscope), which can reduce the load to be imposed on the operator. In addition, the centesis with the puncture needle, for example, may be performed by the operator with one hand operating the operating portion and the other hand operating the puncture needle. Even in this case, a two-dimensional ultrasonic image rendering the puncture needle can be displayed without the necessity for the assistant.

[Appendix 4]

The ultrasonic diagnosis apparatus according to Appendix 2,
wherein the manual selecting means includes a foot switch for inputting the selection by the foot.
In this case, since a tomographic plane may be selected and inputted by the foot, a two-dimensional ultrasonic image to be displayed on the display apparatus can be selected by grasping the ultrasonic probe with both hands even when the ultrasonic probe is of the type to be generally grasped by both hands (such as an ultrasonic endoscope), which can reduce the load to be imposed on the operator. In addition, the centesis with the puncture needle, for example, may be performed by the operator with one hand operating the operating portion and the other hand operating the puncture needle. Even in this case, a two-dimensional ultrasonic image rendering the puncture needle can be displayed without the necessity for the assistant.

[Appendix 5]

The ultrasonic diagnosis apparatus according to any one of Appendices 1 to 4,
wherein the tomographic plane selecting means is configured to select a tomographic plane from the ultrasonic volume data by designating the angle of rotation about the axis of rotation, which is a straight line through two points designated on the ultrasonic volume data.
In this case, a two-dimensional ultrasonic image rendering the longitudinal axis of the puncture needle can be displayed by defining two designated points on the puncture needle if used, for example, which can reduce the examination time.

[Appendix 6]

The ultrasonic diagnosis apparatus according to Appendix 1, further comprising:

the ultrasonic probe is configured to have a therapeutic device channel through which a therapeutic device can be held;
the ultrasonic probe having:

a therapeutic device to be held through the therapeutic device channel; and
position detecting means for detecting the position of the ultrasonic probe and the position of the therapeutic device,

wherein the tomographic plane selecting means selects the tomographic plane based on the position information detected by the position detecting means.
In this case, since the tomographic plane selecting means selects a tomographic plane based on the position information detected by the position detecting means, the two-dimensional ultrasonic image rendering the longitudinal axis of the therapeutic device can be automatically displayed at all times. Thus, the necessity for manual positioning can be eliminated, and the load on the operator and the examination time can be reduced.

[Appendix 7]

The ultrasonic diagnosis apparatus according to Appendix 6, comprising:

the position detecting means having a magnetic sensor;

the therapeutic device having:

a puncture needle having a hollow part to be stuck into a living body; and

a stylet to be held within the hollow part of the puncture needle; and

the multiple magnetic sensors each of which is provided in the ultrasonic probe and the stylet.

In this case, since the magnetic sensor is provided in the stylet, a two-dimensional ultrasonic image rendering the longitudinal axis of the puncture needle can be automatically displayed at all times even when the puncture needle to be used has a hollow part in which it is difficult to provide the magnetic sensor.

[Appendix 8]

The ultrasonic diagnosis apparatus according to Appendix 6 or 7,
wherein the tomographic plane selecting means is used for selecting the tomographic plane based on the position information detected by the position detecting means and further has manual selecting means for inputting the selection of a tomographic plane manually.
In this case, since the automatic selection of a tomographic plane based on position information and the manual selection of a tomographic plane can be performed, the puncture needle can be securely rendered by fine adjustments even when an operation for moving the puncture needle having released the stylet, for example, within the affected part in order to improve the efficiency of cell or tissue extraction. Furthermore, the puncture needle can be securely rendered by fine adjustments even when the accuracy of the sensor decreases due to the poor surrounding atmosphere of the magnetic field.

[Appendix 9]

The ultrasonic diagnosis apparatus according to any one of Appendices 1 to 8,
wherein the ultrasonic probe has multiple ultrasonic transducers aligned two-dimensionally.
In the ultrasonic probe having multiple ultrasonic transducers aligned two-dimensionally, a tomographic plane may be selected from ultrasonic volume data during a scanning operation, and the selected tomographic plane may be displayed as a two-dimensional ultrasonic image.

[Appendix 10]

The ultrasonic diagnosis apparatus according to Appendix 1,
wherein the ultrasonic probe is an ultrasonic probe which can be inserted to a body cavity for use.
In the ultrasonic probe to be inserted to a body cavity for use, a tomographic plane may be selected from ultrasonic volume data during a scanning operation, and the selected tomographic plane may be displayed as a two-dimensional ultrasonic image.

[Appendix 11]

The ultrasonic diagnosis apparatus according to any one of Appendices 1 to 5,
wherein the ultrasonic probe has a therapeutic device channel through which a therapeutic device can be held.
In the ultrasonic probe having a therapeutic device channel through which a therapeutic device can be held, a tomographic plane may be selected from ultrasonic volume data during a scanning operation, and the selected tomographic plane may be displayed as a two-dimensional ultrasonic image.
Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

**Claims**

1.  An ultrasonic diagnosis apparatus comprising:

- an ultrasonic probe (1) for three-dimensionally scanning ultrasonic waves in a living body, the ultrasonic probe (1) including an ultrasonic transducer (16) and a therapeutic device channel (17) in which a therapeutic device (9, 44) is insertable;
- the therapeutic device (9, 44) for insertion into the therapeutic device channel (17);
- position detecting means (7, 8, 41, 42, 43) for detecting a position of the distal end part (13) of the ultrasonic probe (1) and a position of the distal tip of the therapeutic device (9, 44);
- volume data creating means (2) for creating ultrasonic volume data based on an ultrasonic signal captured by the ultrasonic probe (1);
- tomographic plane selecting means (2, 4, 19) for selecting a tomographic plane from the ultrasonic volume data, based on information on the position of the end part (13) of the ultrasonic probe (1) and information on the position of the tip of the therapeutic device (9, 44) detected by the position detecting means (7, 8, 41, 42, 43); and
- a display device (3, 3a) for displaying the tomographic plane selected by the tomographic plane selecting means (2, 4, 19) as a two-dimensional ultrasonic image (32) during the scanning operation.

2. The ultrasonic diagnosis apparatus according to claim 1,
   **characterized in that**

   - the position detecting means (7, 8, 41, 42, 43) further detects an orientation of the end part (13) of the ultrasonic probe (1) and an orientation of the tip of the therapeutic device (9, 44), and that
   - the tomographic plane selecting means is configured to select, from the ultrasonic volume data, a tomographic plane which includes a position vector and a direction vector and is perpendicular to a transducer plane, based on the position vector and the direction vector of the tip of the therapeutic device (9, 44), the position vector and direction vector being obtained based on information on the position and orientation of the end part (13) of the ultrasonic probe (1) and information on the position and orientation of the tip of the therapeutic device (9, 44).

3. The ultrasonic diagnosis apparatus according to claim 2,
   **characterized in that**

   - the position detecting means includes one coil (41) disposed at the tip of the therapeutic device such that a direction of an axis of wiring agrees with a direction of an axis of the therapeutic device, and
   - the information on the position and orientation of the tip of the therapeutic device (9, 44) is of a position vector of the coil (41) and a direction vector in the direction of the axis of the wiring of the coil (41).

4. The ultrasonic diagnosis apparatus according to Claim 1,
   **characterized in that** the tomographic plane selecting means has manual selecting means (4a, 4b, 4c, 4d, 4e) for inputting the selection of a tomographic plane manually.

5. The ultrasonic diagnosis apparatus according to Claim 4,
   **characterized in that** the ultrasonic probe (1) has an operating portion (12) for operations by the hand; and
   the manual selecting means (19) is provided in the operating portion of the ultrasonic probe.

6. The ultrasonic diagnosis apparatus according to Claim 4,
   **characterized in that** the manual selecting means includes a foot switch for inputting the selection by the foot.

7. The ultrasonic diagnosis apparatus according to Claim 1,
   **characterized in that** the tomographic plane selecting means is configured to select a tomographic plane from the ultrasonic volume data by designating the angle of rotation about the axis of rotation, which is a straight line through two points designated on the ultrasonic volume data.

8. The ultrasonic diagnosis apparatus according to Claim 1,
   **characterized in that**

   - the position detecting means having a magnetic sensor (8);
   - the therapeutic device (9) having:
   - a puncture needle having a hollow part configured to be stuck into a living body; and.
   - a stylet (44) configured to be held within the hollow part of the puncture needle; and
   - multiple magnetic sensors (41, 42, 43) each of which being provided in the ultrasonic probe and the stylet (44).

9. The ultrasonic diagnosis apparatus according to Claim 1 or 8.
   **characterized in that** the tomographic plane selecting means is configured to be used for selecting the tomographic plane based on the position information detected by the position detecting means and further has manual selecting means for inputting the selection of a tomographic plane manually.

10. The ultrasonic diagnosis apparatus according to any one of Claims 1 to 9,
    **characterized in that** the ultrasonic probe (1) has multiple ultrasonic transducers (16) aligned two-dimensionally.

11. The ultrasonic diagnosis apparatus according to Claim 1,
    **characterized in that** the ultrasonic probe (1) is configured to be inserted into a body cavity.

**Patentansprüche**

1. Ultraschalldiagnosevorrichtung aufweisend:

   eine Ultraschallsonde (1) zum dreidimensionalen Scannen von Ultraschallwellen in einem lebendigen Körper, wobei die Ultraschallsonde (1) einen Ultraschallwandler (16) und einen Therapieeinheit-Kanal (17) aufweist, in dem eine Therapieeinheit (9, 44) einführbar ist;
   die Therapieeinheit (9, 44) zum Einführen in den Therapieeinheit-Kanal (17);
Positionserfassungsmittel (7, 8, 41, 42, 43) zum Erfassen einer Position des distalen Endteils (13) der Ultraschallsonde (1) und einer Position der distalen Spitze der Therapieeinheit (9, 44); Volumendaten-Generierungsmittel (2) zum Generieren von Ultraschallvolumendaten basierend auf einem durch die Ultraschallsonde (1) erfassten Ultraschallsignal;
Tomographieebene-Auswählmittel (2, 4, 19) zum Auswählen einer Tomographieebene aus den Ultraschallvolumendaten basierend auf durch das Positionserfassungsmittel (7, 8, 41, 42, 43) erfasste Information über die Position des Endteils (13) der Ultraschallsonde (1) und Information über die Position der Spitze der Therapieeinheit (9, 44); und
eine Anzeigeeinheit (3, 3a) zum Anzeigen der durch das Tomographieebene-Auswählmittel (2, 4, 19) ausgewählten Tomographieebene als ein zweidimensionales Ultraschallbild (32) während des Scanvorgangs.

2. Ultraschalldiagnosevorrichtung nach Anspruch 1, **dadurch gekennzeichnet, dass**
das Positionserfassungsmittel (7, 8, 41, 42, 43) ferner eine Ausrichtung des Endteils (13) der Ultraschallsonde (1) und eine Ausrichtung der Spitze der Therapieeinheit (9, 44) erfasst, und dass
das Tomographieebene-Auswählmittel dazu ausgebildet ist, aus den Ultraschallvolumendaten eine Tomographieebene auszuwählen, die einen Positionsvektor und einen Richtungsvektor aufweist und die senkrecht zu einer Wandlerebene ist, basierend auf dem Positionsvektor und dem Richtungsvektor der Spitze der Therapieeinheit (9, 44), wobei der Positionsvektor und der Richtungsvektor basierend auf Information über die Position und Ausrichtung des Endteils (13) der Ultraschallsonde (1) und Information über die Position und Ausrichtung der Spitze der Therapieeinheit (9, 44) erhalten werden.

3. Ultraschalldiagnosevorrichtung nach Anspruch 2, **dadurch gekennzeichnet, dass**
das Positionserfassungsmittel eine an der Spitze der Therapieeinheit angeordnete Spule (41) aufweist, sodass eine Richtung einer Verdrahtungsachse mit einer Richtung einer Achse der Therapieeinheit übereinstimmt, und
die Information über die Position und Ausrichtung der Spitze der Therapieeinheit (9, 44) ein Positionsvektor der Spule (41) und ein Richtungsvektor in der Richtung der Verdrahtungsachse der Spule (41) ist.

4. Ultraschalldiagnosevorrichtung nach Anspruch 1, **dadurch gekennzeichnet, dass** das Tomographieebene-Auswählmittel manuelle Auswählmittel (4a, 4b, 4c, 4d, 4e) zum manuellen Eingeben der Auswahl einer Tomographieebene aufweist.

5. Ultraschalldiagnosevorrichtung nach Anspruch 4, **dadurch gekennzeichnet, dass** die Ultraschallsonde (1) einen Bedienabschnitt (12) für Bedienungen von Hand aufweist; und
das manuelle Auswählmittel (19) in dem Bedienabschnitt der Ultraschallsonde vorgesehen ist.

6. Ultraschalldiagnosevorrichtung nach Anspruch 4, **dadurch gekennzeichnet, dass** das manuelle Auswählmittel einen Fußschalter zum Eingeben der Auswahl durch den Fuß aufweist.

7. Ultraschalldiagnosevorrichtung nach Anspruch 1, **dadurch gekennzeichnet, dass** das Tomographieebene-Auswählmittel dazu ausgebildet ist, eine Tomographieebene aus den Ultraschallvolumendaten durch Bestimmen des Drehwinkels um die Rotationsachse auszuwählen, die eine gerade Linie durch zwei über die Ultraschallvolumendaten bestimmte Punkte ist.

8. Ultraschalldiagnosevorrichtung nach Anspruch 1, **dadurch gekennzeichnet, dass**
das Positionserfassungsmittel einen Magnetsensor (8) aufweist;
die Therapieeinheit (9) umfasst:

eine Punktionsnadel mit einem hohlen Teil, das dazu ausgebildet ist, in einen lebendigen Körper eingestochen zu werden; und
ein Stylet (44), das dazu ausgebildet ist, innerhalb des hohlen Teils der Punktionsnadel gehalten zu werden; und

mehrere Magnetsensoren (41, 42, 43), von denen jeder in der Ultraschallsonde und dem Stylet (44) vorgesehen ist.

9. Ultraschalldiagnosevorrichtung nach Anspruch 1 oder 8,
**dadurch gekennzeichnet, dass** das Tomographieebene-Auswählmittel dazu ausgebildet ist, zum Auswählen der Tomographieebene basierend auf der durch das Positionserfassungsmittel erfassten Positionsinformation verwendet zu werden und ferner manuelle Auswählmittel zum manuellen Eingeben der Auswahl der Tomographieebene aufweist.

10. Ultraschalldiagnosevorrichtung nach einem der Ansprüche 1 bis 9,
**dadurch gekennzeichnet, dass** die Ultraschallsonde (1) mehrere zweidimensional ausgerichtete Ultraschallwandler (16) aufweist.

11. Ultraschalldiagnosevorrichtung nach Anspruch 1, **dadurch gekennzeichnet, dass** die Ultraschallsonde (1) dazu ausgebildet ist, in eine Körperhöhle eingeführt zu werden.

## Revendications

1. Appareil de diagnostic à ultrasons comprenant :

    - une sonde à ultrasons (1) destinée à balayer en trois dimensions des ondes ultrasonores dans un corps vivant, la sonde à ultrasons (1) comprenant un transducteur à ultrasons (16) et un canal de dispositif thérapeutique (17) dans lequel un dispositif thérapeutique (9, 44) peut être inséré ;
    - le dispositif thérapeutique (9, 44) pour insertion dans le canal de dispositif thérapeutique (17) ;
    - un moyen de détection de position (7, 8, 41, 42, 43) destiné à détecter une position de la partie d'extrémité distale (13) de la sonde à ultrasons (1) et une position de la pointe distale du dispositif thérapeutique (9, 44) ;
    - un moyen de création de données en volume (2) destiné à créer des données en volume ultrasonores sur la base d'un signal ultrasonore capturé par la sonde à ultrasons (1) ;
    - un moyen de sélection de plan tomographique (2, 4, 19) destiné à sélectionner un plan tomographique à partir des données en volume ultrasonores, sur la base des informations sur la position de la partie d'extrémité (13) de la sonde à ultrasons (1) et des informations sur la position de la pointe du dispositif thérapeutique (9, 44) détectées par le moyen de détection de position (7, 8, 41, 42, 43) ; et
    - un dispositif d'affichage (3, 3a) destiné à afficher le plan tomographique sélectionné par le moyen de sélection de plan tomographique (2, 4, 19) en tant qu'image ultrasonore bidimensionnelle (32) pendant l'opération de balayage.

2. Appareil de diagnostic à ultrasons selon la revendication 1, **caractérisé en ce que**

    - le moyen de détection de position (7, 8, 41, 42, 43) détecte en outre une orientation de la partie d'extrémité (13) de la sonde à ultrasons (1) et une orientation de la pointe du dispositif thérapeutique (9, 44), et **en ce que**
    - le moyen de sélection de plan tomographique est configuré pour sélectionner, à partir des données en volume ultrasonores, un plan tomographique qui inclut un vecteur de position et un vecteur de direction et est perpendiculaire à un plan du transducteur, sur la base du vecteur de position et du vecteur de direction de la pointe du dispositif thérapeutique (9, 44), le vecteur de position et le vecteur de direction étant obtenus sur la base des informations sur la position et l'orientation de la partie d'extrémité (13) de la sonde à ultrasons (1) et des informations sur la position et l'orientation de la pointe du dispositif thérapeutique (9, 44).

3. Appareil de diagnostic à ultrasons selon la revendication 2, **caractérisé en ce que**

    - le moyen de détection de position comprend une bobine (41) disposée au niveau de la pointe du dispositif thérapeutique d'une manière telle qu'une direction d'un axe de câblage s'accorde avec une direction d'un axe du dispositif thérapeutique, et
    - les informations sur la position et l'orientation de la pointe du dispositif thérapeutique (9, 44) correspondent à un vecteur de position de la bobine (41) et un vecteur de direction dans le sens de l'axe du câblage de la bobine (41).

4. Appareil de diagnostic à ultrasons selon la revendication 1, **caractérisé en ce que** le moyen de sélection de plan tomographique comporte un moyen de sélection manuelle (4a, 4b, 4c, 4d, 4e) destiné à délivrer en entrée la sélection d'un plan tomographique manuellement.

5. Appareil de diagnostic à ultrasons selon la revendication 4, **caractérisé en ce que** la sonde à ultrasons (1) comporte une partie opérationnelle (12) pour les opérations manuelles ; et le moyen de sélection manuelle (19) est prévu dans la partie opérationnelle de la sonde à ultrasons.

6. Appareil de diagnostic à ultrasons selon la revendication 4, **caractérisé en ce que** le moyen de sélection manuelle comprend un commutateur à pied destiné à délivrer en entrée la sélection avec le pied.

7. Appareil de diagnostic à ultrasons selon la revendication 1, **caractérisé en ce que** le moyen de sélection de plan tomographique est configuré pour sélectionner un plan tomographique à partir des données en volume ultrasonores en désignant l'angle de rotation autour de l'axe de rotation, qui est une ligne droite à travers deux points désignés sur les données en volume ultrasonores.

8. Appareil de diagnostic à ultrasons selon la revendication 1, **caractérisé en ce que**

- le moyen de détection de position comporte un capteur magnétique (8) ;
- le dispositif thérapeutique (9) comporte :

  - une aiguille de piquage comportant une partie creuse configurée pour être enfoncée dans un corps vivant ; et
  - un stylet (44) configuré pour être maintenu à l'intérieur de la partie creuse de l'aiguille de piquage ; et

  - de multiples capteurs magnétiques (41, 42, 43) chacun étant prévu dans la sonde à ultrasons et le stylet (44).

9. Appareil de diagnostic à ultrasons selon la revendication 1 ou 8, **caractérisé en ce que** le moyen de sélection de plan tomographique est configuré pour être utilisé pour sélectionner le plan tomographique sur la base des informations de position détectée par le moyen de détection de position et comporte en outre un moyen de sélection manuelle destiné à délivrer en entrée la sélection d'un plan tomographique manuellement.

10. Appareil de diagnostic à ultrasons selon l'une quelconque des revendications 1 à 9, **caractérisé en ce que** la sonde à ultrasons (1) comporte de multiples transducteurs à ultrasons (16) alignés de façon bidimensionnelle.

11. Appareil de diagnostic à ultrasons selon la revendication 1, **caractérisé en ce que** la sonde à ultrasons (1) est configurée pour être insérée dans une cavité de corps.

# FIG.1

*1:* ULTRASONIC ENDOSCOPE

*11:* INSERTION PORTION

*12:* OPERATING PORTION

*13:* END PART

*14:* BENDING PART

*15:* FLEXIBLE PART

*16:* ULTRASONIC TRANSDUCER

*19:* TWO-DIMENSIONAL IMAGE SELECT KNOB

*2:* ULTRASONIC OBSERVING APPARATUS

*3* MONITOR

TRANSMISSION DRIVE SIGNAL

ULTRASONIC IMAGE SELECT SIGNAL

ULTRASONIC WAVE

*17a*

*17:* FORCEPS CHANNEL

*17b*

ECHO SIGNAL

*18:* BEND KNOB

ULTRASONIC TRANSMITTING PORTION *22*

ULTRASONIC RECEIVING PORTION *23*

*21*

COMPUTING/ CONTROL PORTION

ULTRASONIC IMAGE CREATING PORTION *24*

DISPLAY CONTROL PORTION *25*

*4c:* SECOND-POINT DESIGNATE KEY

*4b:* FIRST-POINT DESIGNATE KEY

*4e:* TWO-DIMENSIONAL IMAGE SELECT KEY (–)

*4a:* SCAN START KEY

*4d:* TWO-DIMENSIONAL IMAGE SELECT KEY (+)

*4:* KEYBOARD

*5:* TRACK BALL

EP 1 804 079 B1

# FIG.2

16a: SIGNAL LINE

TRANSMISSION DRIVE SIGNAL

13: END PART

TO ULTRASONIC OBSERVING APPARATUS 2

16: ULTRASONIC TRANSDUCER

ECHO SIGNAL

ULTRASONIC SCANNING RANGE

17a: FORCEPS CHANNEL PORT

9: PUNCTURE NEEDLE

# FIG.3

16a: SIGNAL LINE

TRANSMISSION DRIVE SIGNAL

13: END PART

TO ULTRASONIC OBSERVING APPARATUS 2

16: ULTRASONIC TRANSDUCER

ECHO SIGNAL

17a: FORCEPS CHANNEL PORT

9: PUNCTURE NEEDLE

# FIG.4

3a: MONITOR SCREEN

32: TWO-DIMENSIONAL
ULTRASONIC IMAGE

9: PUNCTURE
NEEDLE

31: 3D DATA

# FIG.5

3a: MONITOR SCREEN

32: TWO-DIMENSIONAL
ULTRASONIC IMAGE

9a: PUNCTURE NEEDLE
BASAL PART

FIRST POINT

31: 3D DATA

33: CURSOR

EP 1 804 079 B1

# FIG.6

3a: MONITOR SCREEN

32: TWO-DIMENSIONAL ULTRASONIC IMAGE

9b: PUNCTURE NEEDLE TIP PART

SECOND POINT

31: 3D DATA

33: CURSOR

# FIG.7

3a: MONITOR SCREEN

32: TWO-DIMENSIONAL ULTRASONIC IMAGE

9: PUNCTURE NEEDLE

31: 3D DATA

# FIG.8

1: ULTRASONIC ENDOSCOPE

11: INSERTION PORTION   12: OPERATING PORTION

19: TWO-DIMENSIONAL IMAGE SELECT KNOB

3

MONITOR

13: END PART  14: BENDING PART  15: FLEXIBLE PART

2: ULTRASONIC OBSERVING APPARATUS

TRANSMISSION DRIVE SIGNAL

ULTRASONIC IMAGE SELECT SIGNAL

43: THIRD TRANSMITTING COIL

42: SECOND TRANSMITTING COIL

ULTRASONIC TRANSMITTING PORTION   22

16: ULTRASONIC TRANSDUCER

17a  9,44

ULTRASONIC RECEIVING PORTION   23

21

41: FIRST TRANSMITTING COIL

MAGNETIC FIELD

17b

ECHO SIGNAL

18: BEND KNOB

17: FORCEPS CHANNEL

6b: - KEY

6a: + KEY

ULTRASONIC IMAGE CREATING PORTION

COMPUTING/CONTROL PORTION

8: RECEIVING COIL

24   25

DISPLAY CONTROL PORTION

COIL EXCITING SIGNAL

7

POSITION/ORIENTATION DETECTING DEVICE

6: FOOT SWITCH

POSITION/ORIENTATION DATA

5: TRACK BALL

4c: SECOND-POINT DESIGNATE KEY

4f: AUTOMATIC DETECTION KEY

4b: FIRST-POINT DESIGNATE KEY

4a: SCAN START KEY

4e: TWO-DIMENSIONAL IMAGE SELECT KEY (-)

4d: TWO-DIMENSIONAL IMAGE SELECT KEY (+)

4A: KEYBOARD

EP 1 804 079 B1

21

# FIG.9

44: STYLET

9: PUNCTURE NEEDLE

41: FIRST TRANSMITTING COIL

**EP 1 804 079 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005058584 A
- JP 2000185041 A
- JP 2004208859 A
- WO 9625881 A1